# FASCICULE DE BREVET EUROPEEN

(11) **EP 0 709 074 B1**
(45) Date de publication et mention de la délivrance du brevet: **18.12.2002**
(21) Numéro de dépôt: 95420298.2
(22) Date de dépôt: 27.10.1995
(51) Int. Cl.: A61F 2/38

(54) **Elément prothétique tibial pour prothèse du genou**
Schienbeinprothesenteil für Knieprothese
Tibial-prosthetic part for knee prosthesis

(30) Priorité: 27.10.1994 FR 9413092
(43) Date de publication de la demande: 01.05.1996
(73) Titulaire: Merck Biomaterial France, 26903 Valence Cedex 09 (FR); Bascoulergue, Gérard, F-62520 Le Touquet (FR); Charret, Philippe, 69270 Fontaine sur Saone (FR); Fayard, Jean-Philippe, F-42170 St. Just St. Rambert (FR); Hulin, Paul Henri, 89000 Auxerre (FR); Peyrot, Jacques, 69160 Tassin la Demi Lune (FR)
(72) Inventeur: Bascoulergue, Gérard, F-62520 Le Touquet (FR); Charret, Philippe, F-69270 Fontaine sur Saone (FR); Dupré-Latour, Laurent, F-42580 L'Etrat (FR); Fayard, Jean-Philippe, F-42170 St Just St Rambert (FR); Hulin, Paul Henri, F-89000 Auxerre (FR); Peyrot, Jacques, F-69160 Tassin la Demi Lune (FR); Collomb, Jean, F-26800 Portes les Valence (FR)
(74) Mandataire: Ropital-Bonvarlet, Claude

(56) Documents cités:
- EP-A- 0 304 219
- DE-U- 9 100 292
- DE-U- 9 300 791
- FR-A- 2 663 536

## Description

La présente invention concerne le domaine des prothèses osseuses et vise, plus particulièrement, celui des prothèses du genou, qu'elles soient uni ou pluricompartimentales.

Dans le domaine technique ci-dessus, il est connu depuis longtemps déjà de proposer des systèmes articulaires artificiels visant à remplacer l'articulation naturelle, constituée par la conformation épiphysaire basse du fémur, par la conformation épiphysaire complémentaire haute du tibia et par l'élément fémoro-patellaire.

Toutes les propositions connues jusqu'à présent, qu'elles soient implantées couramment ou non, s'appuient sur une constitution d'un tel système articulaire artificiel à partir de deux éléments principaux, destinés à être respectivement adaptés, après résection osseuse, sur la conformation épiphysaire basse du fémur et sur la conformation épiphysaire complémentaire haute du tibia.

L'invention concerne plus spécifiquement les propositions relatives aux éléments d'articulation destinés à être implantés sur la conformation épiphysaire haute du tibia.

La conformation anatomique de l'épiphyse haute du tibia fournit une contrepartie complémentaire aux condyles fémoraux, de manière à disposer d'une complémentarité de surfaces articulaires aptes à assurer la fonction complexe de flexion-extension avec rotation interne ou externe partielle du genou.

Dans ce but, l'articulation naturelle dispose dans le plateau tibial de deux glènes associées à des ménisques et dans lesquelles prennent appui, sous le contrôle de deux ligaments croisés et de deux groupes de ligaments latéraux, les surfaces condyliennes de l'épiphyse basse du fémur.

Lors de l'implantation d'une prothèse, il est nécessaire de pratiquer une résection plus ou moins importante du plateau tibial pour substituer à l'une ou aux deux glènes de ce plateau, des éléments artificiels destinés à assurer la même fonction vis-à-vis des condyles fémoraux.

En règle générale, une telle fonction est assumée en implantant après résection, un élément prothétique tibial comprenant une embase destinée à être adaptée sur la coupe tibiale par des moyens appropriés qui peuvent être de simples vis, une sorte de prolongement court ou encore une queue longue introduite dans le canal médullaire avec ou sans présence de ciment intermédiaire. Une telle embase est destinée à supporter un patin généralement en matière plastique, tel qu'en polyéthylène, offrant, par sa face supérieure, la surface nécessaire à l'appui du ou des condyles fémoraux.

Le certificat d'utilité allemand DE 93 00 791 décrit, par exemple, un élément prothétique tibial dont l'embase est adaptée au moyen de plusieurs vis et d'une queue longue, portant des nervures, introduite dans le canal médullaire.

L'implantation de l'embase par l'intermédiaire d'une ou plusieurs vis n'apparaît pas entièrement satisfaisante car des fatigues ou nécroses osseuses, ainsi que des jeux ou tolérances d'usinage ou d'usure, sont généralement responsables d'un déplacement relatif possible de cette embase par rapport aux condyles fémoraux.

S'agissant d'une articulation complexe telle que le genou, dans laquelle il peut être considéré qu'aucun déplacement ne peut être qualifié de pur, une telle possibilité de déplacement relatif devrait être évitée.

L'implantation d'une prothèse tibiale artificielle par l'intermédiaire d'un prolongement court, telle qu'elle peut être considérée à partir de l'enseignement fourni par la publication de la demande FR 2 663 536, n'est pas considérée comme à même de résoudre le problème précédent car la liaison intime avec la masse osseuse ne peut pas être appréciée, sur la base des connaissances actuelles, comme susceptible d'apporter une bonne immobilisation dans le plan sagittal, mais aussi dans le plan frontal, de même que dans les plans intermédiaires. C'est à tel point vrai que, selon cette demande et semble-t-il pour réduire les contraintes imposées à l'ancrage à queue courte, il est préconisé d'adapter le patin sur l'embase en réservant un degré de liberté relative du patin autorisant un débattement rotatoire.

Quant à l'implantation d'un élément prothétique tibial au moyen d'une queue longue, les problèmes liés à une telle intervention lourde sont connus et ont fait l'objet de commentaires sur les inconvénients qui s'y attachent concernant la solidité de l'ancrage, la bio-compatibilité du ciment et le caractère important de la résection qu'il convient de pratiquer pour une telle implantation.

Tous les inconvénients ci-dessus sont certainement largement à considérer dans le cas d'implantation d'un élément prothétique faisant partie d'une prothèse pluricompartimentale, mais sont notablement plus pénalisants lorsqu'il s'agit de procéder à l'implantation d'une prothèse unicompartimentale. En effet, dans un tel cas, la partie du plateau tibial concernée se trouve limitée à l'un des massifs latéraux qui offre, pour la bonne implantation et la bonne immobilisation d'un élément prothétique unicompartimental, une surface et une masse largement plus faibles lorsqu'il convient de prendre en compte supplémentairement que de telles interventions doivent laisser subsister l'insertion des ligaments croisés ou au moins l'un deux.

L'objet de l'invention est de proposer un élément prothétique tibial pour prothèse du genou, du type comprenant un patin de coopération avec les condyles fémoraux et une embase pourvue, sur sa face inférieure, de moyens d'insertion sur la partie haute du tibia et, sur sa face inférieure, de moyens de liaison avec le patin:
- les moyens d'insertion comprenant au moins :
   . une lame rigide s'étendant perpendiculairement au plan de l'embase et à partir de la face inférieure de cette dernière, en étant orientée parallèlement mais à distance du plan sagittal,
   . une conformation, associée à ladite lame et saillant à partir de ladite face,
- les moyens de liaison comprenant :
   . un trou pratiqué pour que son axe coïncide avec le barycentre de la conformation et s'ouvrant au niveau de la face supérieure de l'embase pour l'engagement d'un téton formé par le patin,
   . au moins une barrette rigide s'élevant à partir de la face supérieure de l'embase en étant disposée dans le prolongement de la lame rigide pour coopérer avec une partie complémentaire du patin et assurer l'immobilisation angulaire de celui-ci.

Bien que plus particulièrement dirigés vers un élément prothétique tibial constitutif d'une prothèse unicompartimentale, les moyens de l'invention trouvent une application élargie en étant adaptables à tous les éléments prothétiques tibiaux.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est une perspective illustrant les moyens de l'invention en relation avec une représentation schématique des épiphyses fémorale et tibiale concernées.

La **fig. 2** est une vue en plan prise selon le plan **II-II** de la **fig. 1.**

La **fig. 3** est une perspective en vue de dessous de l'élément selon la **fig. 2.**

La **fig. 4** est une variante illustrant un développement de l'élément prothétique selon la **fig. 3.**

La **fig. 5** est une vue en plan montrant une autre forme de réalisation de l'élément suivant la **fig. 4.**

La **fig. 6** est une perspective représentant un développement de l'objet de l'invention.

La **fig. 7** est une vue latérale partie en coupe d'une autre forme de réalisation de l'élément prothétique.

La **fig. 8** est une vue en plan prise selon la ligne **VIII-VIII** de la **fig. 7.**

La **fig. 9** est une vue latérale partie en coupe montrant, à plus grande échelle, un avantage de la disposition selon la **fig. 7.**

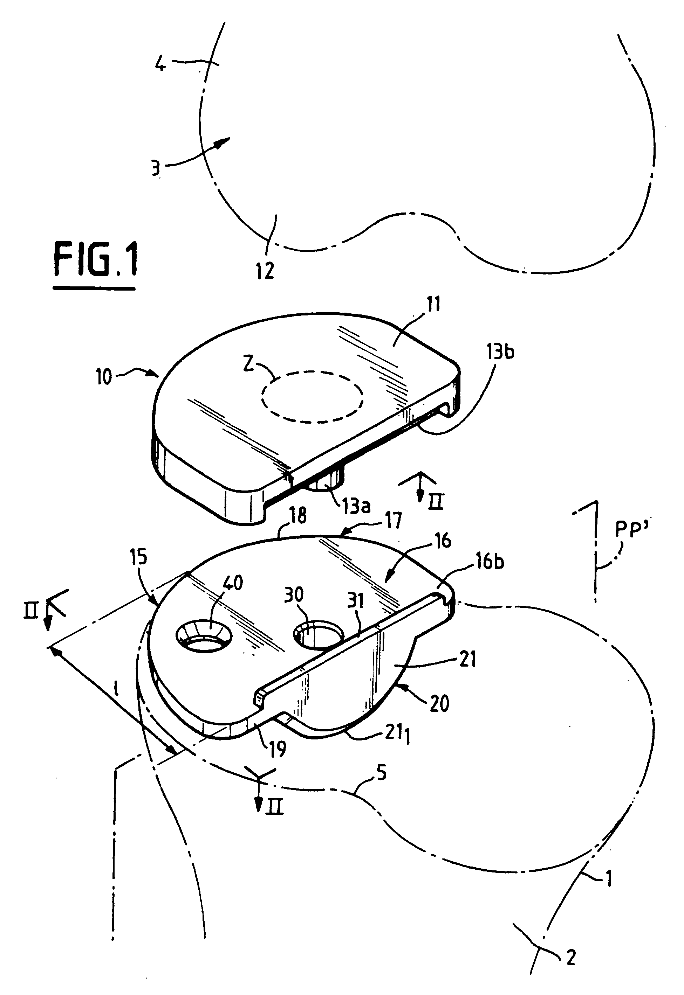

La **fig. 1** montre un exemple de réalisation de l'élément prothétique tibial selon l'invention. Un tel élément est illustré comme faisant partie d'une prothèse de genou unicompartimentale et devant être implanté sur l'épiphyse haute **1** d'un tibia **2,** de manière à offrir une contrepartie fonctionnelle au condyle **3** d'un fémur **4** associé.

Selon l'illustration présentée, l'élément prothétique s'analyse comme étant une adaptation gauche interne ou droite externe, une réalisation symétrique par rapport à un plan devant être considérée pour une adaptation gauche externe et droite interne. Le caractère symétrique par rapport à un plan de référence doit ici être considéré en relation avec un plan sagittal **P-P'** passant par le massif épineux **5** séparant les deux glènes d'un plateau tibial naturel, non réséqué.

Dans cette forme de réalisation selon les figures **1** à **3**, l'élément prothétique comprend un patin **10** réalisé en une matière plastique appropriée, telle qu'en polyéthylène, et destiné à offrir, par sa surface supérieure **11**, une zone d'appui **Z** au condyle fémoral complémentaire **12.** A cette fin, la surface supérieure **11** peut être plane ou présenter une cavité de réception.

Le patin **10** est pourvu de moyens **13a et 13b** de liaison avec une embase porteuse **15** destinée à être implantée ou insérée sur le massif tibial **1** après résection du plateau tibial. L'embase **15** est réalisée sous la forme d'une platine **16** épaisse, en toute matière appropriée et, plus particulièrement, en métal. La platine présente en plan une forme en demi-lune délimitée par un bord périphérique **17** comportant une partie **18** courbe et une partie **19** rectiligne. La partie **19** est destinée à être orientée, lors de la pose ou de l'adaptation, en direction du plan sagittal **P-P'** et généralement à distance de ce dernier de manière à préserver l'intégrité des ligaments croisés, dans le cas d'application à une prothèse unicompartimentale. Dans ce but, la largeur **l** de la platine est toujours inférieure à la mesure prise entre le plan sagittal **P-P'** et un plan fictif parallèle tangent à la surface extérieure du massif latéral épiphysaire correspondant à la glène concernée.

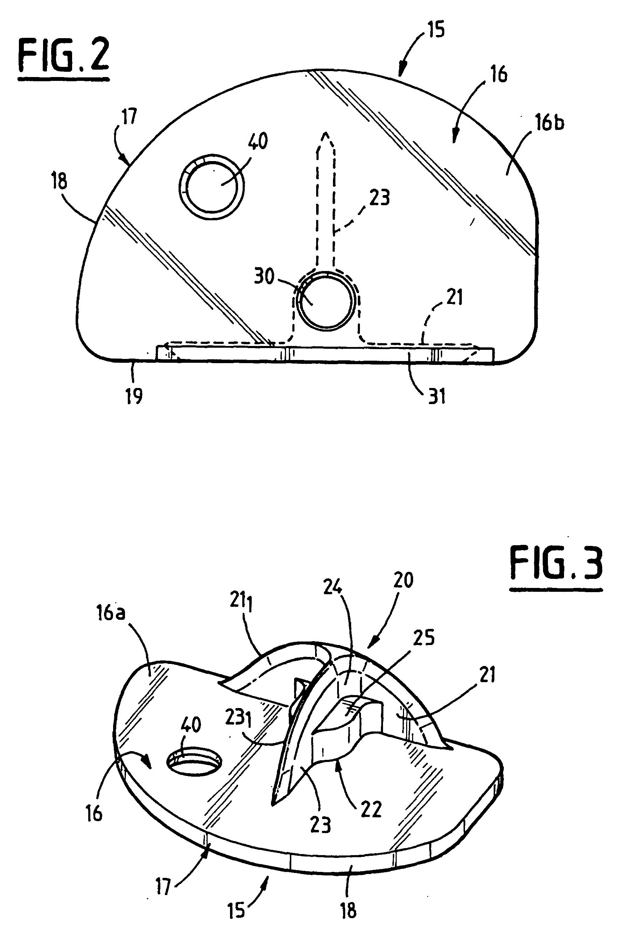

La platine **16** présente sur sa face inférieure **16a,** tel que cela ressort des figures **2** et **3,** des moyens **20** d'insertion dans la masse osseuse de l'épiphyse **1.** Ces moyens d'insertion comprennent, dans la forme d'exécution représentée par les figures **1** à **3**, tout d'abord une lame **21** rigide, établie parallèlement et dans le prolongement de la partie rectiligne **19** du bord **17** et délimitée par une arête extrême courbe **21**_{**1**}. La lame **21** présente, de préférence, à sa base et dans le plan passant par la partie **19,** une longueur inférieure à cette dernière.

Les moyens **20** comprennent, par ailleurs, une conformation **22** saillant à partir de la face inférieure **16a** de la platine **16** et faisant partie intégrante avec cette dernière. La conformation **22** comprend, dans l'exemple illustré, une seconde lame **23** qui s'étend, en direction de la partie **18** du bord **17,** depuis la lame **21** en présentant une même hauteur maximale que cette dernière à laquelle elle est raccordée par des congés **24.** La lame **23** possède une arête courbe **23**_{**1**} et son raccordement avec la lame **21** confère à l'ensemble une forme pseudo-cruciforme.

La conformation **22** est complétée par des massifs **25** qui occupent les angles rentrants délimités par le raccordement de la lame **23** avec la lame **21,** de tels massifs **25** étant solidaires, au vrai sens du terme, des lames et de la platine **16.**

L'embase **15** comporte également des moyens de liaison avec le patin **10.** De tels moyens de liaison comprennent un trou **30** qui est pratiqué, dans l'exemple présenté, à partir de la surface supérieure plane **16b** de la platine **16,** selon un axe perpendiculaire à la platine en étant centré sur un axe général coïncidant avec le barycentre de la conformation **22**, pour correspondre, en quelque sorte, à la masse la plus importante de cette conformation constituée à partir de la face inférieure **16a** par les massifs **25,** les congés **24** et l'épaisseur de la lame **23.** Le trou **30** est pratiqué sur une profondeur inférieure à la hauteur des massifs **25.** Le trou **30** est, par ailleurs, pratiqué pour présenter un diamètre interne compatible avec l'insertion, de préférence à force, du téton **13** présenté par le patin **10.**

Les moyens de liaison portés par l'embase **15** comprennent, par ailleurs, une barrette 31 rigide, s'élevant à partir de la face supérieure plane **16b** pour coopérer avec une partie complémentaire du patin, afin d'immobiliser angulairement ce dernier. Selon une forme de réalisation avantageuse, la barrette **31** est ménagée uniquement le long de la partie **19** dans le prolongement de cette dernière, ainsi que dans celui de la lame **21,** le reste du bord périphérique de la platine étant dépourvu de tout rebord saillant par rapport à la surface **16b.** La barrette **31** est destinée à coopérer avec la partie rectiligne **13b** du pourtour ou de la périphérie du patin **10,** laquelle partie peut être formée par un évidement de conformation complémentaire à la barrette **31** pour emboîter cette dernière.

Outre les moyens ci-dessus, il est prévu de ménager, dans l'embase **15,** un trou **40,** de préférence fraisé de façon excentrée, à partir de la face supérieure **16b,** de manière à permettre l'engagement et le passage d'une vis de fixation osseuse, amenée à pénétrer à l'intérieur de l'épiphyse **1**.

L'adaptation ou l'implantation de l'élément prothétique tibial décrit ci-dessus, consiste à assurer la résection du compartiment concerné de l'épiphyse **1,** de manière à supprimer la masse osseuse devant être remplacée par une adaptation artificielle.

La résection peut être effectuée pour délimiter une zone d'appui plane sur laquelle l'embase **15** est implantée par enfoncement des moyens **20** et **22** dans la masse osseuse avec ou sans interposition d'une couche de ciment interface.

L'enfoncement est rendu possible par la forme des lames **21** et **23,** ainsi que par leurs dispositions relatives qui permettent de ménager un accueil réceptif convenable pour les massifs **25** de la conformation **22.**

Une telle implantation, qui sera contrainte, dans son application, contre l'épiphyse **1**, par l'action des ligaments, mais aussi par la pression qui sera exercée en utilisation normale par le poids du corps du sujet appareillé, est par ailleurs assurée de façon stable, sans possibilité de déplacement relatif, en raison de l'aspect cruciforme des moyens **20** et **22** qui permettent de garantir, par la lame **21,** une immobilisation dans le plan sagittal et, par la lame **23,** une immobilisation dans le plan frontal.

Ces moyens s'opposent également à toute possibilité de déplacement angulaire relatif. Un complément de fixation est assuré par la mise en place ou l'implantation d'une vis osseuse à travers le trou **40.**

Lorsque l'adaptation de l'embase **15** a été effectuée, il suffit d'emboîter le patin **10,** en assurant l'engagement du téton **13a** dans le trou **30,** de façon à amener en coopération mutuelle la partie complémentaire **13b** avec la barrette **31.** De tels moyens assurent une immobilisation angulaire du patin **10** sur l'embase **15.**

L'élément prothétique tibial décrit ci-dessus doit être considéré, en relation avec la **fig. 1,** comme présentant une largeur **l** qui est inférieure à l'étendue frontale correspondante de la masse épiphysaire unicompartimentale du tibia **2,** d'une mesure suffisante pour tenir compte du passage et de l'insertion des ligaments croisés qui en aucun cas ne doivent être concernés par l'implantation dans la masse osseuse de la lame **21**.

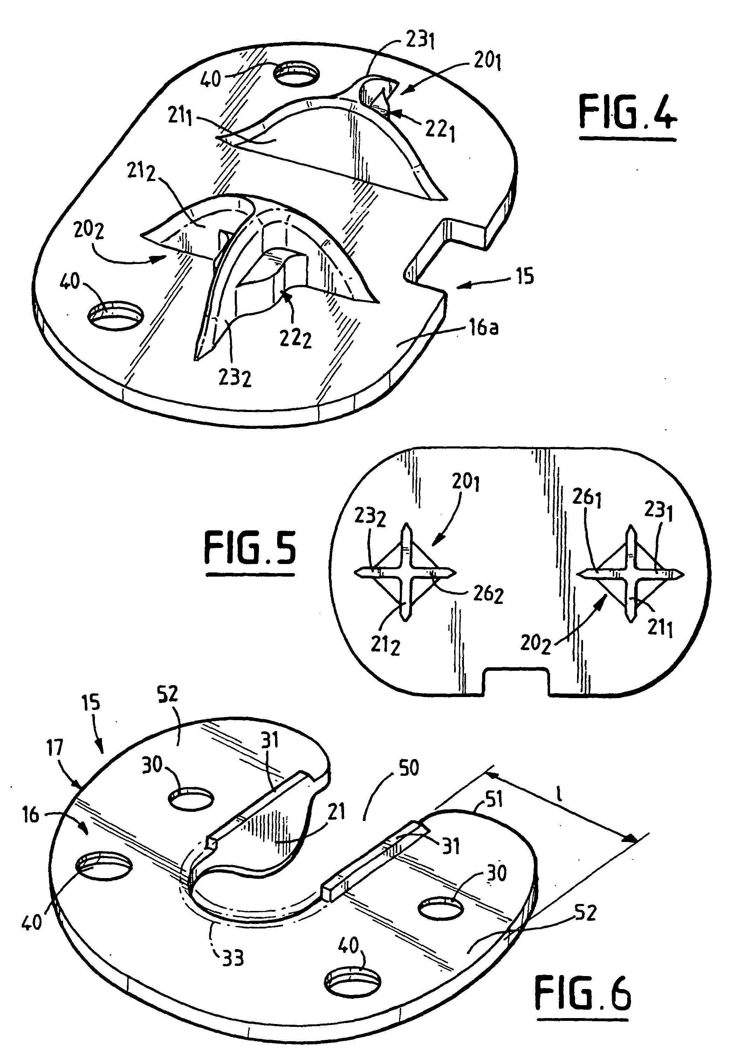

Les dispositions ci-dessus, qui concernent plus particulièrement un élément prothétique faisant partie d'une prothèse de genou unicompartimentale, peuvent être transposées à un élément prothétique tibial pour une prothèse de genou pluricompartimentale comme cela est illustré par la **fig. 4.**

Dans un tel cas, il peut être prévu de faire venir sur la surface inférieure **16a** de la platine **16,** deux ensembles de moyens **20**_{**1**}**-20**_{**2**} et **22**_{**1**}**-22**_{**2**} répondant chacun aux caractéristiques structurelles décrites en référence à la **fig. 3.** Dans un tel cas et avec les réserves précédemment données, les lames **21**_{**1**} et **21**_{**2**} s'étendent parallèlement et à distance l'une à l'autre en se faisant face.

Il peut être prévu selon la **fig. 5** d'adjoindre à chaque lame **21**_{**1**} et **21**_{**2**}, une contre-lame **26**_{**1**}, **26**_{**2**}, s'étendant dans le même plan, mais à l'opposé de la lame **23**_{**1**}**, 23**_{**2**}**.**

La **fig. 6** montre une variante de mise en oeuvre des moyens selon l'invention dans le cas d'un élément prothétique tibial faisant partie d'une prothèse pluricompartimentale, laissant subsister les ligaments croisés. Dans un tel cas, la platine **16** présente, en plan, une forme sensiblement en U définie par un dégagement **50** pratiqué depuis la partie **51** postérieure du bord périphérique de l'embase **15**. Le dégagement **50** délimite ainsi deux branches **52** dont les bords internes sont pourvus chacun d'une barrette **31** assurant l'immobilisation angulaire d'un patin de forme en plan complémentaire de celle de la platine. Les barrettes **31** peuvent, le cas échéant, être réunies par un prolongement **33** suivant exactement le contour du dégagement **50.**

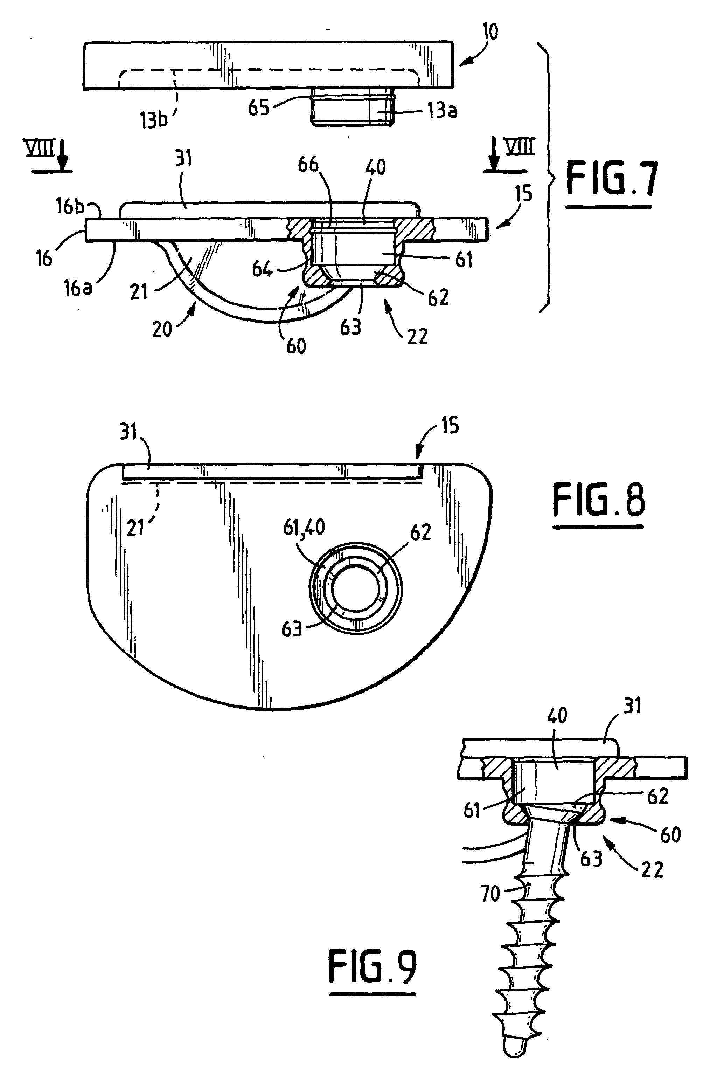

Les **fig. 7** et **8** montrent un développement selon lequel l'embase **15** comporte des moyens **20** comprenant la lame **21** qui répond aux mêmes caractéristiques que celles précédemment énoncées. Dans cet exemple, outre la lame **21,** les moyens **20** comprennent, par ailleurs, une conformation **22** qui est constituée sous la forme d'un bossage cylindrique tubulaire **60** formé en saillie à partir de la face **16a** de la platine, en étant coaxial au trou **40.** Le bossage **60** est exécuté pour comporter dans le prolongement du trou **40,** un puits **61** de même diamètre dont le fond communique avec une portée sphérique annulaire **62** dont la grande base est d'un diamètre inférieur à celui du fond du puits. La petite base de la portée **62** est prolongée par un trou **63,** de préférence à paroi annulaire divergente s'ouvrant à l'extérieur du bossage **60.** Bien que cela ne soit pas obligatoire, le bossage **60** présente une paroi périphérique extérieure pourvue d'une conformation **64** de type annelée ou analogue.

Dans cet exemple, le bossage **60** associé au trou **4** permet d'assurer les fonctions suivantes.

Tout d'abord, lors de l'adaptation de l'embase **15,** le bossage **60** assume une fonction de pion d'ancrage par son insertion dans la masse osseuse et vient ainsi compléter la fonction de la lame **21.**

Ensuite, de façon facultative ou non, le bossage **60** permet la pose d'une vis de fixation **70,** telle que présentée par la **fig. 9.** Une telle vis peut en effet être engagée dans le puits **61,** à travers le trou **40,** pour que sa tête prenne appui sur la portée **62.** En choisissant, au sens de l'invention, de pratiquer le trou **63** avec un diamètre plus grand que celui du corps de vis, cette dernière peut alors être orientée selon une inclinaison souhaitée dans les limites de la plage d'orientation autorisée pour adapter la fixation recherchée en fonction des exigences d'implantation.

Il convient de noter que la profondeur de la portée **62** est choisie pour loger complètement la tête de vis, quelle que soit l'orientation de la vis, afin de laisser libre tout le volume utile du puits **61** et du trou **40.**

Enfin, la présence coaxiale du trou **40** et du puits **61** permet de délimiter un logement de bonnes dimensions utiles, apte à recevoir un téton **13a** qui est prévu dans cet exemple pour être décalé par rapport à la position occupée dans les exemples précédents. Le téton **13a** peut comporter dans cet exemple une moulure périphérique **65** favorisant son immobilisation lors de l'engagement dans le trou **40** qui présente avantageusement une rainure **66.**

Il va de soi que les dispositions selon les figures **7** à **9** peuvent être adaptées à des éléments prothétiques à caractère pluricompartimental.

L'invention n'est pas limitée aux exemples décrits et représentés car diverses modifications peuvent y être apportées sans sortir de son cadre.

## Revendications

1. Elément prothétique tibial pour prothèse du genou, du type comprenant un patin **(10)** de coopération avec le condyle fémoral associé **(3)** et une embase **(15)** pourvue, sur sa face intérieure, de moyens d'insertion, sur la partie épiphysaire haute du tibia et sur sa face supérieure, de moyens de liaison avec le patin,
- les moyens d'insertion comprenant au moins :
. une lame rigide **(21)** s'étendant perpendiculairement au plan de l'embase et à partir de la face inférieure **(16a)** de cette dernière, en étant orientée parallèlement mais à distance du plan sagittal **(P-P')**,
. une conformation **(22)** associée à ladite lame et saillant à partir de ladite face inférieure,
- les moyens de liaison comprennent :
. au moins un trou **(30)** pratiqué pour que son axe coïncide avec le barycentre de la conformation **(22)** et s'ouvrant au niveau de la face supérieure **(16b)** de l'embase pour l'engagement d'un téton **(13a)** présenté par le patin,
. au moins une barrette rigide **(31)** s'élevant à partir de la face supérieure de l'embase en étant disposée dans le prolongement de la lame rigide **(21)** pour coopérer avec une partie complémentaire **(13b)** du patin et assurer l'immobilisation angulaire de ce dernier.

2. Elément prothétique selon la revendication **1**, correspondant à une prothèse du type unicompartimental, la lame **(21)** s'étendant dans le prolongement d'une partie rectiligne **(19)** du bord périphérique **(17)** de l'embase, laquelle partie rectiligne est orientée, lors de la pose, en direction du plan sagittal **(P-P')** de la prothèse.

3. Elément prothétique selon la revendication **1** ou **2**, l'embase présentant, par son bord périphérique, une partie rectiligne **(19)** dans le seul prolongement duquel s'élève la barrette **(31)** pour coopérer avec une partie complémentaire **(13b)** du bord périphérique du patin.

4. Elément prothétique selon la revendication **1**, correspondant à une prothèse du type total et comportant deux conformations **(22)** formées par la face inférieure **(16a)** de l'embase **(15)** pour être situées sensiblement à l'aplomb de zones d'appui **(Z)** des condyles fémoraux, en position d'extension sensiblement totale du tibia par rapport à ce dernier.

5. Elément prothétique selon la revendication **4**, la conformation **(22)** comprenant une lame rigide **(23)** s'étendant perpendiculairement à la lame **(21)** à laquelle elle est raccordée et des massifs solides (**25**) occupant les angles rentrants délimités par les lames.

6. Elément prothétique selon la revendication **5**, le trou **(30)** étant ménagé dans l'axe général coïncidant avec le barycentre de la conformation **(22)** formée par la lame **(23)** et les massifs **(25)**.

7. Elément prothétique selon la revendication **4**, la conformation **(22)** étant du type cruciforme.

8. Elément prothétique selon la revendication la revendication **1**, **2** ou **4**, la conformation **(22)** étant formée par un bossage **(60)** présenté en saillie par la face inférieure **(16a)** de l'embase dans l'axe d'un trou **(40)** s'ouvrant dans la face supérieure **(16b)** de façon décentrée par rapport à l'embase et destinée à recevoir le téton **(13a)**.

9. Elément prothétique selon la revendication **8**, le bossage étant percé axialement pour présenter un puits **(61)** prolongeant le trou **(40)**, une portée annulaire sphérique **(62)** dont la grande base coïncide avec le fond du puits en présentant un diamètre inférieur et un trou traversant **(63)** défini par une paroi divergente à partir de la petite base de la portée.

10. Elément prothétique selon la revendication **9**, la profondeur de la portée annulaire sphérique étant choisie pour recevoir entièrement la tête d'une vis de fixation osseuse **(70)** alors que le diamètre du trou (**63**) étant choisi supérieur à celui de la vis.

11. Elément prothétique selon la revendication **9**, le puits **(61)** présentant un même diamètre que le trou **(40)** pour recevoir un téton **(13a)**.

12. Elément prothétique selon la revendication **8**, le bossage (**60**) présentant une conformation **(64)** adaptée de sa surface périphérique extérieure.

13. Elément prothétique selon l'une des revendications **1** à **12**, l'embase présentant en plan une forme sensiblement en U définie par un dégagement **(50)** ménagé à partir du bord postérieur **(51)** de l'embase et réservé au passage des ligaments croisés, un tel dégagement délimitant dans le plateau deux branches **(52)** dont les bords internes sont pourvus chacun d'une barrette **(31)** constitutive des moyens de liaison et dont la surface inférieure **(16a)** est munie de moyens **(21 et 22)**.

14. Elément prothétique selon la revendication **13**, les barrettes des bords internes des deux branches de l'embase étant raccordées par un prolongement frontal **(33)**.

## Patentansprüche

1. Tibiale Prothesenkomponente für eine Kniegelenkprothese mit einem Gleitelement (10) zum Zusammenwirken mit der zugehörigen Femurkondyle (3) und einer Auflage (15), die an der Unterseite mit Mitteln zum Einsetzen am oberen Epiphyse-Teil der Tibia und an der Oberseite mit Mitteln zum Verbinden mit dem Gleitelement versehen ist,
- wobei die Mittel zum Einsetzen wenigstens umfassen:
. eine starre Zunge (21), die sich von der Unterseite (16a) der Auflage ausgehend senkrecht zur Ebene der Auflage erstreckt und hierbei parallel zur Sagittalebene (P-P'), jedoch von dieser beabstandet, ausgerichtet ist,
. eine Ausbildung (22), die dieser Zunge zugehörig ist und an der Unterseite vorsteht,
- wobei die Mittel zum Verbinden umfassen:
. wenigstens ein Loch (30), das so gearbeitet ist, dass seine Achse deckungsgleich mit dem Schwerpunkt der Ausbildung (22) ist, und das sich zum Einführen eines am Gleitelement angeordneten Zapfens (13a) an der Oberseite (16b) der Auflage öffnet,
. wenigstens eine starre Leiste (31), die sich an der Oberseite der Auflage erhebt und in der Verlängerung der starren Zunge (21) angeordnet ist, um mit einem dazu komplementären Teil (13b) des Gleitelements zusammenzuwirken und dessen seitliche Fixierung zu gewährleisten.

2. Prothesenkomponente nach Anspruch 1,
als eine unikondyläre Prothese, wobei die Zunge (21) in der Verlängerung eines geraden Teils (19) des Umfangsrands (17) der Auflage verläuft und das gerade Teil beim Einsetzen zur Sagittalebene (P-P') der Prothese ausgerichtet ist.

3. Prothesenkomponente nach Anspruch 1 oder 2,
wobei die Auflage an ihrem Umfangsrand ein gerades Teil (19) aufweist, an dem sich ausschließlich die Leiste (31) erhebt, um mit einem dazu komplementären Teil (13b) des Umfangsrands des Gleitelements zusammenzuwirken.

4. Prothesenkomponente nach Anspruch 1,
als eine Totalprothese mit zwei Ausbildungen (22), die an der Unterseite (16a) der Auflage (15) gebildet sind und im Wesentlichen rechtwinklig zu den Auflagebereichen (Z) der Femurkondylen angeordnet sind, wenn sich die Tibia in Bezug auf diese im Wesentlichen in einer vollständig gestreckten Haltung befindet.

5. Prothesenkomponente nach Anspruch 4,
wobei die Ausbildung (22) eine starre Zunge (23) umfasst, die senkrecht zu der Zunge (21) verläuft, an der sie angesetzt ist, sowie massive Sockel (25), die in den von den Zungen begrenzten zurückspringenden Winkeln liegen.

6. Prothesenkomponente nach Anspruch 5,
wobei das Loch (30) in der allgemeinen Achse angeordnet ist, die mit dem Schwerpunkt der von der Zunge (23) und den Sockeln (25) gebildeten Ausbildung (22) zusammenfällt.

7. Prothesenkomponente nach Anspruch 4,
wobei die Ausbildung (22) kreuzförmig ist.

8. Prothesenkomponente nach Anspruch 1, 2 oder 4,
wobei die Ausbildung (22) durch eine Vorwölbung (60) gebildet ist, die an der Unterseite (16a) der Auflage in der Achse eines Lochs (40) vorsteht, welches sich an der Oberseite (16b) in Bezug auf die Auflage seitlich versetzt öffnet und zum Aufnehmen des Zapfens (13a) dient.

9. Prothesenkomponente nach Anspruch 8,
wobei die Vorwölbung axial durchbohrt ist, um einen das Loch (40) verlängernden Schacht (61), und eine ringförmige sphärische Aufnahme (62) aufzuweisen, deren große Basis mit dem Boden des Schafts deckungsgleich ist und einen kleineren Durchmesser als dieser aufweist, sowie eine durchgehende Bohrung (63), die von einer von der kleinen Basis der Aufnahme ausgehenden auseinander strebenden Wandung gebildet ist.

10. Prothesenkomponente nach Anspruch 9,
wobei die Tiefe der ringförmigen sphärischen Aufnahme so gewählt ist, dass der Kopf einer Knochenbefestigungsschraube (70) vollständig aufgenommen werden kann, wogegen der Durchmesser der Bohrung (63) so gewählt ist, dass er größer ist als der der Schraube.

11. Prothesenkomponente nach Anspruch 9,
wobei der Schacht (61) den gleichen Durchmesser wie das Loch (40) zum Aufnehmen des Zapfens (13a) aufweist.

12. Prothesenkomponente nach Anspruch 8,
wobei die Vorwölbung (60) eine Ausbildung (64) aufweist, die deren äußerer Umfangsfläche angepasst ist.

13. Prothesenkomponente nach einem der Ansprüche 1 bis 12,
wobei die Auflage in der Ebene im Wesentlichen die Form eines U aufweist, das durch eine Ausnehmung (50) gebildet ist, die vom rückwärtigen Rand (51) der Auflage ausgehend angeordnet und für die Durchführung der Kreuzbänder vorgesehen ist, wobei eine derartige Ausnehmung an der Platte zwei Arme (52) begrenzt, deren Innenränder jeweils mit einer zu den Verbindungsmitteln gehörenden Leiste (31) versehen sind und deren Unterseite (16a) mit Mitteln (21 und 22) versehen ist.

14. Prothesenkomponente nach Anspruch 13,
wobei die Leisten an den Innenrändern der beiden Arme der Auflage durch eine stirnseitige Verlängerung (33) miteinander verbunden sind.

## Claims

1. Tibial prosthetic element for a knee prosthesis of the type consisting of a connection block (10) for the corresponding femoral condyle (3) and a baseplate (15) equipped, on its inner side, with means for insertion into the upper epiphyseal part of the tibia and, on its outer side, means for linking to the block,
- means for insertion comprised of at least:
. a rigid blade (21) extending perpendicularly to the baseplate and from the lower side (16a) of the latter, being oriented parallel to, but away from, the sagittal plane (P-P')
. a conformation (22) connected to said blade and projecting from said inner side,
- means for connection comprised of:
. at least one hole (30) such that its axis is in line with the barycentre of the conformation (22) and opening at the outer side (16b) of the baseplate in order to lock a stud (13a) present on the block.
. at least one rigid bar (31) arising from the outer side of the baseplate and positioned along the length of the rigid blade (21) to function with a complementary section (13b) of the block in order to ensure angular immobilisation of the latter.

2. Prosthetic element according to claim 1 corresponding to a single-compartment type prosthesis, with the blade (21) extending along the same line as a rectilinear part (19) of the peripheral edge (17) of the blade, said rectilinear part being oriented in the in the sagittal plane (P-P') of the prosthesis.

3. Prosthetic element according to claim 1 or 2 whose baseplate has a rectilinear part (19) along its edge only along the same line as the bar (31) to function with a complementary part (13b) of the peripheral edge of the block.

4. Prosthetic element according to claim 1 corresponding to a total prosthesis with two conformations (22) formed by the lower side (16a) of the baseplate (15) to be situated directly above the support zones (Z) of the femoral condyles, in a position of nearly complete extension of the tibia with respect to the latter.

5. Prosthetic element according to claim 4 wherein the conformation (22) includes a rigid blade (23) which extends perpendicularly to the blade (21) to which it is attached and solid ridges (25) occupying the re-entrant angles formed by the blades.

6. Prosthetic element according to claim 5 wherein the hole (30) is positioned along the general axis of the barycentre of the conformation (22) formed by the blade (23) and the ridges (25).

7. Prosthetic element according to claim 4 wherein the conformation (22) is shaped like a cross.

8. Prosthetic element according to claim 1, 2 or 4 wherein the conformation (22) is formed by a protrusion (60) projecting from the inner side (16a) of the baseplate along the axis of a hole (40) opening on the outer side (16b), off-centre with respect to the baseplate, and into which the stud (13a) locks.

9. Prosthetic element according to claim 8 wherein the protrusion is pierced axially to give wells (61) along the line of the hole (40), a spherical annular area of bearing (62) whose main baseplate lines up with the bottom of the wells by means of a smaller diameter and a cross-hole (63) defined by a wall divergent from the small baseplate of the area of bearing.

10. Prosthetic element according to claim 9 wherein the depth of the spherical annular area of bearing is chosen to receive the entire head of a bone-clamping screw (70) whereas the diameter of the cross-hole (63) is such that it is bigger than that of the screw.

11. Prosthetic element according to claim 9 wherein the wells (61) have the same diameter as the hole (40) for the stud (13a).

12. Prosthetic element according to claim 8 wherein the protrusion (60) has an adapted conformation (64) of its external peripheral surface.

13. Prosthetic element according to one of claims 1 to 12 wherein the baseplate has a U-shape delimited by an opening (50) positioned on the posterior side (51) of the baseplate and reserved for cross ligaments, this outlet delimiting two sections (52) in the plate whose inner edges are each equipped with a bar (31) constituting means of connection and whose lower side (16a) is equipped with means (21 and 22).

14. Prosthetic element according to claim 13 wherein the bars on the inner edges of the two sections of the baseplate are connected by a frontal extension (33).
